# EUROPÄISCHE PATENTSCHRIFT

(11) **EP 3 484 562 B1**
(45) Veröffentlichungstag und Bekanntmachung des Hinweises auf die Patenterteilung: **17.02.2021**
(21) Anmeldenummer: 17777771.1
(22) Anmeldetag: 13.07.2017
(51) Int. Cl.: A61M 11/00, A61M 15/00

(54) **VERNEBLEREINHEIT MIT WECHSELBAREM MUNDSTÜCK**
ATOMIZER UNIT COMPRISING A REPLACEABLE MOUTHPIECE
UNITÉ DE NÉBULISATION À EMBOUT REMPLAÇABLE

(30) Priorität: 14.07.2016 DE 102016112990
(43) Veröffentlichungstag der Anmeldung: 22.05.2019
(73) Patentinhaber: NEBU-TEC med. Produkte Eike Kern GmbH, 63820 Elsenfeld (DE)
(72) Erfinder: KERN, Stefan, 63820 Elsenfeld (DE)
(74) Vertreter: Pöhner, Wilfried Anton
(86) Internationale Anmeldenummer: PCT/DE2017/100582
(87) Internationale Veröffentlichungsnummer: WO 2018/010734

(56) Entgegenhaltungen:
- EP-A1- 3 000 499
- JP-A- H05 337 183
- US-A1- 2014 230 811
- US-A1- 2016 121 057

## Beschreibung

Vorliegende Erfindung beschäftigt sich mit einer Verneblereinheit mit wechselbarem Mundstück gemäß des Oberbegriffs des Anspruchs 1. Zur Behandlung von Erkrankungen der Atemwege bietet sich häufig eine Inhalationstherapie an, bei der eine Medikamentenlösung durch einen Vernebler zu einem Aerosol aus feinen, mikrometerdurchmessenden Tröpfchen zerstäubt wird. Der Vorteil einer Verabreichung in dieser Form ist, dass das Medikament direkt an seinen Einsatzort gebracht wird, was zum einen schneller und auch effizienter ist als ein Transport durch die Blutbahn.

Für eine Inhalationstherapie eingesetzte Vernebler umfassen üblicherweise ein Verneblereinheit mit Mundstück, ein Medikamentenreservoir und eine Steuereinheit, welche mit der Verneblereinheit verbunden wird und die den in der Verneblereinheit vorhandenen Aerosolerzeuger steuert. Der Aerosolerzeuger ist bei modernen Verneblern aus einer metallenen Meshmembran und einem Schwingungserzeuger, sowie eventuell einem mechanischen Kopplungsteil in Form einer Trägerplatte aufgebaut. Die Meshmembran ist ein dünnes Metallblech, welches in einem mittleren Bereich feine, das heißt mikrometergroße, trichterförmige Poren aufweist, wobei der größere Querschnitt des Trichters zum Reservoir hin weist. Der Schwingungserzeuger ist in der Regel ein kreisringförmiger Piezokristall, welcher mit der Meshmembran, eventuell mittels einer dazwischen geschalten Trägerplatte, mechanisch gekoppelt ist und dabei den perforierten Bereich umgibt. Elektrische Anregung und dadurch bewirkte Verformung des Piezokristalls bewirkt eine Verbiegung des perforierten Bereichs der Meshmembran, welche in einem durch das elektrische Anregungssignal vorgegebenen Takt schwingt, wodurch eine auf einer Zuführungsseite anliegende Medikamentenflüssigkeit in die feinen Poren gedrückt und auf der Ausgangsseite in Form eines Aerosols aus mikrometerfeinen Tröpfen abgegeben wird.

Damit ein Anwender diese Verneblereinheit auch verwenden kann, muss ein Mundstück angeformt oder aufgesetzt sein, dessen vorderes Ende so gestaltet ist, dass es mit den Lippen einfach umschließbar ist, sodass ein luftdichter oder weitestgehend luftdichter Abschluss herstellbar ist. Somit wird es dem Anwender ermöglicht, beim Einatmen Luft durch das ausgangsseitig des Aerosolerzeugers innerhalb der Verneblereinheit und dem Mundstück vorhandenen Volumen einzusaugen, welches sich mit dem dort erzeugten Aerosol vermischt und idealer Weise bis in die Bronchien oder die Lunge gelangt. Damit der Abtransport funktionieren kann, müssen in dem ausgangsseitig des Aerosolerzeugers vorhandenen Volumen Lufteinlasslöcher vorhanden sein. Diese sind üblicherweise bei dem Stand der Technik verfügbaren Verneblern in der Verneblereinheit selber vorhanden.

Der Nachteil hierbei ist, dass dadurch einerseits die Reinigung erschwert wird, und andererseits die eingezogene Luftmenge bzw. der zum Einsaugen der vorgegebenen Luftmenge nötige Druck nicht leicht einstellbar ist. Letzteres ist für den Inhalationskomfort eines Anwenders wichtig.

Das Dokument US 2016/0121057 A1 offenbart einen Vernebler mit einem wechselbaren Mundstück, welches einen an seiner Unterseite angeformten, senkrecht von einer Längsrichtung des Mundstücks wegweisenden Einatemstiel aufweist. Dieser bildet einen Luftkanal in dem beim Einatmen durch das Mundstück Luft aus der Umgebung in die Aerosolkammer des Verneblers strömt.

In der Schrift US 2014/0230811 A1 wird ebenfalls ein Vernebler mit einem wechselbaren Mundstück beschrieben, wobei im Inneren des Mundstücks eine Unterteilung für die Ausbildung zweier Luftkanäle, eines Medikamentenkanals und eines Ausatemkanals sorgt.

Vorliegende Erfindung stellt sich die Aufgabe, eine Verneblereinheit mit wechselbarem Mundstück zu kreieren, welche eine einfache Reinigung erlaubt und sowohl eine einfache Anpassung des Luftflusses an einen vom Anwender gewünschten oder bei einer Therapie angebrachten Grad als auch eine genauere Überwachung des Atemverlaufs bei der Inhalation gestattet.

Diese Aufgabe wird gelöst durch eine Verneblereinheit mit wechselbarem Mundstück nach Anspruch 1. Ein erstes wesentliches Merkmal ist hierbei, dass das Mundstück wechselbar ausgeführt ist, das heißt es ist mit einem Ende so geformt ist, dass es auf eine Anschlussmöglichkeit an der Verneblereinheit angeschlossen oder aufgesetzt werden kann. Dies ist im einfachsten Fall so gelöst, dass an der erfindungsgemäßen Verneblereinheit aerosolerzeugerausgangsseitig ein zylinderförmiger Stutzen vorhanden ist, auf welchem ein komplementär geformter, hinterer Teil des erfindungsgemäßen Mundstücks aufsteckbar ist. Zylinderform ist deshalb vorgeschlagen, weil sie sich am einfachsten und günstigsten herstellen lässt, jedoch ist jede andere Form des Querschnitts ebenfalls denkbar, so zum Beispiel oval und/oder rechteckig und/oder vieleckig mit oder ohne abgerundeten Ecken.

Ein zweites wesentliches Merkmal des wechselbaren Mundstücks der erfindungsgemäßen Verneblereinheit sind jedoch die Lufteinlasskanäle, welche an ihm angeformt sind. Diese befinden sich bevorzugt an einer Seite des Mundstücks wobei eine, die vordere Öffnung des oder der Kanäle in ein durch das Mundstückanschlussstück der Verneblereinheit sowie dem Mundstück selber gebildeten, ausgangsseitig des Aerosolerzeuger gelegenen Volumen mündet. Das rückwärtige, andere Ende der Luftkanäle ist nach außen offen, sodass dadurch Luft eingesaugt werden kann.

Das hintere Ende eines dieser Kanäle ist an einen zu einem Sensor führenden, an der Außenseite der Verneblereinheit vorhandenen Flow-Kanal angeschlossen.

Der Vorteil der Wechselbarkeit besteht darin, dass für jeden Anwender ein passendes Mundstück bereit gehalten werden kann, welches sowohl in der Form des durch die Lippen ergriffenen Teils, sowie auch in der Größe, d.h. der lichten Weite, der Luftkanäle an die Bedürfnisse des Anwenders angepasst ist. Ein weiterer, wichtiger Vorteil ist die vereinfachte Reinigung des erfindungsgemäßen Aerosolerzeugers, dadurch, dass das abnehmbare Mundstück der aerosolerzeugerausgangsseitige Teil der Verneblereinheit leichter zugänglich wird und das Mundstück selbst, da es keine empfindlichen Teile enthält, einfach abgewaschen beziehungsweise sterilisiert werden kann. Dadurch, dass die Lufteinlasskanäle im Mundstück selber vorhanden sind, wird auch erreicht, dass bei einem eventuellen Ausatmen durch Mundstück und Vernebler weniger Kondensat bis zum Aerosolerzeuger gelangt und diesen kontaminiert. Auch dies trägt zu einer erhöhten Hygiene der erfindungsgemäßen Verneblereinheit mit wechselbarem Mundstück bei.

Die Menge der eingesaugten Luft lässt sich dadurch vorteilhalt leichter einstellen, dass Mundstücke mit verschieden großen Luftkanälen eingesetzt werden. Wenn mehr Luft eingesaugt werden soll, zum Beispiel um das Aerosol stärker zu verdünnen und einen Hustenreiz eines Anwenders nicht aufkommen zu lassen, so lässt sich einfach ein Mundstück mit größeren Öffnungen aufsetzen. Bei Verneblereinheiten, welche eine Öffnung in der Einheit selber aufweisen, ist eine Änderung der eingesaugten Luftmenge nicht so einfach möglich.

Ist wenigstens einer der Luftkanäle der erfindungsgemäßen Verneblereinheit mit Mundstück so gestaltet, dass er mit seinem hinteren Ende bündig mit einem Ende eines zu einem Drucksensor führenden, an der Verneblereinheit vorhandenen Flow-Kanals abschließt. Dadurch wird der effektive Abnahmepunkt der Druckmessung in den mundstückwärtigen Teil der Aerosolkammer verlegt ist, kann ein deutlicheres Signal gemessen und der Atemverlauf des Anwenders bei der Inhalation genauer Überwacht werden.

Soll so ein bündiger Abschluss hergestellt werden, so empfiehl vorliegende Erfindung, dass entweder der Querschnitt des Anschlussstücks sowie des hinteren Teils des Mundstücks keine Symmetrie aufweist, das heißt nicht zylinderförmig ist, oder im Wesentlichen zylinderförmig ist aber im Mundstück oder im Anschlussstück der Verneblereinheit eine Nut sowie in dem jeweils anderen Teil eine dazu komplementäre Kerbe vorhanden ist, sodass das Zusammenstecken immer nur einer bestimmten Ausrichtung erfolgen kann und ein ungewolltes Verdrehen, der Art, dass der zum Flow-Kanal führende Luftkanal des Mundstücks nicht mit diesem fluchtet ausgeschlossen ist.

Vorteilhafte Weiterbildungen vorliegender Erfindung, welche einzeln oder in Kombination realisierbar sind, sofern sie sich nicht gegenseitig offensichtlich ausschließen, sollen im Folgenden beschrieben werden.

In einer einfachsten Ausführung besitzt das Mundstück der erfindungsgemäßen Verneblereinheit einen einzigen Luftkanal mit den oben beschriebenen Eigenschaften. Vorteilhafter Weise können jedoch weitere Luftkanäle vorhanden sein. Insbesondere empfiehlt es sich, drei oder mehr Luftkanäle vorzusehen, weil dadurch selbst dann, wenn einer dieser Luftkanäle zum Anschluss an einen Druck- oder Durchflussmesssensor verwendet wird, noch zwei zum Lufteinsaugen offene Kanäle vorhanden sind. Diese sollten idealerweise so angeordnet sein, dass eine gute Durchmischung der eingesaugten Luft mit dem erzeugten Aerosol gewährleistet ist und ein möglichst vollständiger Abtransport stattfindet. Zum einen aus medizinischen Gründen, da möglichst viel des Medikaments seinen Zielort erreichen soll, und zum anderen ist auch ein hygienischer Vorteil gegeben, da durch den vollständigen Abtransport des Aerosols verhindert wird, dass sich Medikamentenflüssigkeit in der Aerosolkammer ausgangsseitig des Aerosolerzeugers als Kondensat niederschlägt und zu einem erhöhten Reinigungsbedarf führt.

Das an der Verneblereinheit angeformte Aufsatzstück für das Mundstück kann entweder zylinderförmig sein oder auch einen ovalen, elliptischen oder vieleckigen Querschnitt aufweisen. Wird aus Gründen der einfachen Herstellung eine Zylinderform bevorzugt, so schlägt vorliegende Erfindung vor, dass bei Verwendung eines Luftkanals an einen zu einem Druck- oder Durchflusssensor führenden Flow-Kanal der Verneblereinheit anschließenden Luftkanals vermeiden des Aufsetzens mit falscher Orientierung entweder im Anschlussstück oder am Mundstück eine Kerbe und in dem jeweils anderen Teil eine komplementäre Nut angeformt sind.

In einer bevorzugten Ausführungsform schlägt vorliegende Erfindung vor, dass das Mundstück der erfindungsgemäßen Verneblereinheit mehr als einen und insbesondere genau drei Luftkanäle besitzt. Zwei sollen hierbei in symmetrischer Position an je einer Seite des Mundstücks und einer an einer Unterseite gelegen sein. Diese Zahl an Luftkanälen ist deshalb empfehlenswert, weil selbst bei Verwendung eines Kanals, z.B. des auf der Unterseite befindlichen, als Zuleitung für einen Flow-Kanal der Verneblereinheit, noch zwei weitere vorhanden sind, die bei symmetrischer Positionierung eine gute Durchmischung der eingesaugten Luft mit dem in der Aerosolkammer produzierten Aerosol und einen weitgehenden Abtransport des verdünnten Aerosols gewährleisten.

Als bevorzugtes Material des wechselbaren Mundstücks vorliegender Erfindung empfiehlt sich aufgrund seiner günstigen Herstellung und des niedrigen Gewichts sowie der allgemeinen chemischen Beständigkeit, welche eine Reinigung und Sterilisierung des Mundstücks erleichtert, Kunststoff.

Der Luftkanal des Mundstücks vorliegender Erfindung ist bevorzugt mit einem hinteren Ende nach außen offen, kann aber auch bündig abschließend an eine Eingangsöffnung eines am Drucksensor oder Durchflusssensor führenden Flow-Kanals, der beispielsweise an der Unterseite des Gehäuses der erfindungsgemäßen Verneblereinheit angeformt ist, angeschlossen sein. In ersterem Fall erfüllt der Luftkanal seinen bestimmungsgemäßen Hauptzweck, nämlich das Einsaugen von Luft aus dem Außenraum in den aerosolerzeugerausgangsseitigen Innenraum der Verneblereinheit zu ermöglichen und einen Abtransport des erzeugten Aerosols zu gewährleisten. Besonders bevorzugt sind hierbei die Luftkanäle des Mundstücks so gestaltet, dass ihre Innenseite jeweils von der Außenseite des Anschlussstücks der Verneblereinheit gebildet ist. Somit ist im abgenommenen Zustand des Mundstücks jeder Teil des Mundstücks für eine Reinigung leicht zugänglich. Die nur an beiden Enden offenen Luftführungskanäle "entstehen" dann erst beim Aufstecken des Mundstücks auf die erfindungsgemäße Verneblereinheit.

In der erfindungsgemäßen Verneblereinheit selbst können entweder zusätzlich Öffnungen vorhanden sein, die eine stärkere Lufteinsaugung ermöglichen, oder die Aerosolkammer ist so gestaltet, dass sie nur an der zum Mundstück führenden Seite offen ist, sodass der Lufteinlass ausschließlich durch die Luftkanäle des Mundstücks möglich ist.

Die erfindungsgemäße Verneblereinheit besitzt in einer besonders bevorzugten Ausführungsform über ein mittels einer Kappe fest und flüssigkeitsdicht verschließbares Medikamentenreservoir, in das die zu Inhalierende Medikamentenflüssigkeit einfüllbar ist.

Weitere Einzelheiten, Merkmale und Vorteile der Erfindung ergeben sich aus den folgenden, anhand der Figuren näher erläuterten bevorzugten Ausführungsbeispielen. Diese sollen die Erfindung nur illustrieren und in keiner Weise einschränken.

Es zeigen im Einzelnen:
- Fig. 1:: Perspektivische Ansicht einer Ausführungsform des wechselbaren Mundstücks.
- Fig. 2:: Längsschnitt durch eine Ausführungsform der erfindungsgemäßen Verneblereinheit mit aufgesetztem, wechselbarem Mundstück.
- Fig. 3:: In drei Teilfiguren eine Fünfseitenansicht des wechselbaren Mundstücks der erfindungsgemäßen Verneblereinheit.

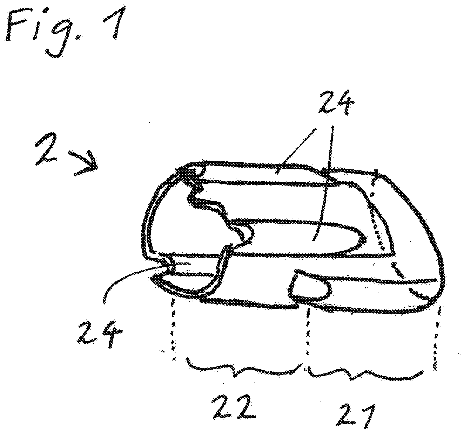

Fig. 1 zeigt in perspektivischer Ansicht ein wechselbares Mundstück 2 vorliegender Erfindung. Wie zu sehen ist, ist das Mundstück 2 im Wesentlichen in zwei Abschnitte 21, 22 gegliedert. Der Vordere 21 weist einen ovalen Querschnitt auf und ist so geformt und dimensioniert, sodass ein Anwender ihn einfach mit den Lippen ergreifen und luftdicht umschließen kann. Dieser vordere Teil 21 geht kontinuierlich in den hinteren Teil 22 über, welcher zum Aufstecken auf das Anschlussstück 12 der Verneblereinheit 1 dient. Dieser hintere Teil 22 weist im Wesentlichen einen Kreisförmigen Querschnitt auf, an dem circa 120 Grad zueinander versetzt Ausbuchtungen 24 mit halbkreisförmigem Querschnitt aufgesetzt sind. Diese sind im nicht aufgesteckten Zustand vollkommen offen und daher für eine Reinigung leicht zugänglich. Wird das Mundstück 2 jedoch auf das zylinderförmige Anschlussstück 12 der Verneblereinheit 1 aufgeschoben oder - gesteckt so sind durch die Außenseite des Anschlussstücks 12 und die Auswölbungen 24 Lufteinlasskanäle 24 gebildet. Somit ist es möglich, die Verneblereinheit 1 derart zu gestalten, dass die aerosolerzeugerausgangseitig vorhandene Aerosolkammer 120 nur an der zum Mundstück führenden Stirnseite offen und ansonsten vollständig geschlossen ist, das heißt keine anderen Lufteinlassöffnungen vorhanden sind.

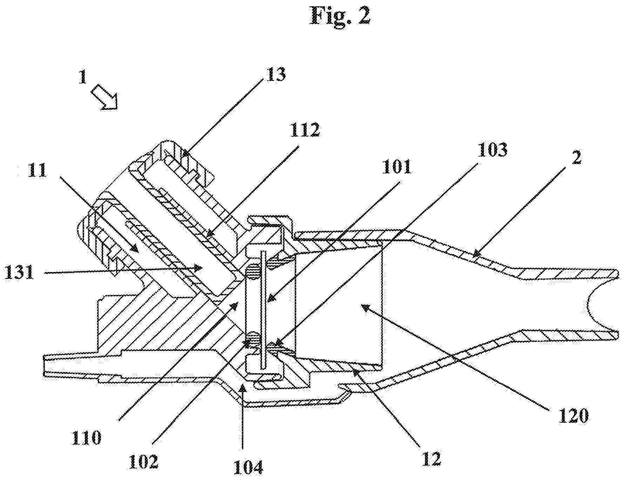

Fig. 2 zeigt einen Längsschnitt durch eine erfindungsgemäße Verneblereinheit 1 mit aufgesetztem Mundstück mit Luftkanälen. Das Anschlussstück 12 auf welches das Mundstück 2 aufgesetzt ist, hat einen zum Innendurchmesser des Mundstücks komplementär kreisförmigen Querschnitt. An der Unterseite der Verneblereinheit ist ein Flow-Kanal 104 angebracht, welcher am hinteren Ende mit einem Stutzen endet, welcher an eine Steuereinheit (hier nicht gezeigt) anschließbar ist. Sein vorderes Ende schließt im aufgesetzten Zustand des Mundstücks 2 bündig mit einem der Luftkanäle 24 des Mundstücks 2 ab, wodurch ein verlustfreies Messen der eingesaugten Luftmenge und damit des Atemverlaufs ermöglicht ist. Das gemessene Signal ist bei der hier vorgeschlagenen Ausgestaltung weiterhin dadurch vorteilhaft verstärkt, dass die Ausgangsöffnung des an den Flow-Kanal 104 angeschlossenen Luftkanals 24 in dem im Mundstück gelegenen Teil der Aerosolkammer 120 gelegen ist. Dieser vorverlegte Druckabnahmepunkt erfährt eine größere Druckvariation im Verlauf eines Atemzyklus, da der Luftstrom dort schneller und somit der dynamische Anteil des Gesamtdruckes dort großer ist, bzw. stärker variiert.

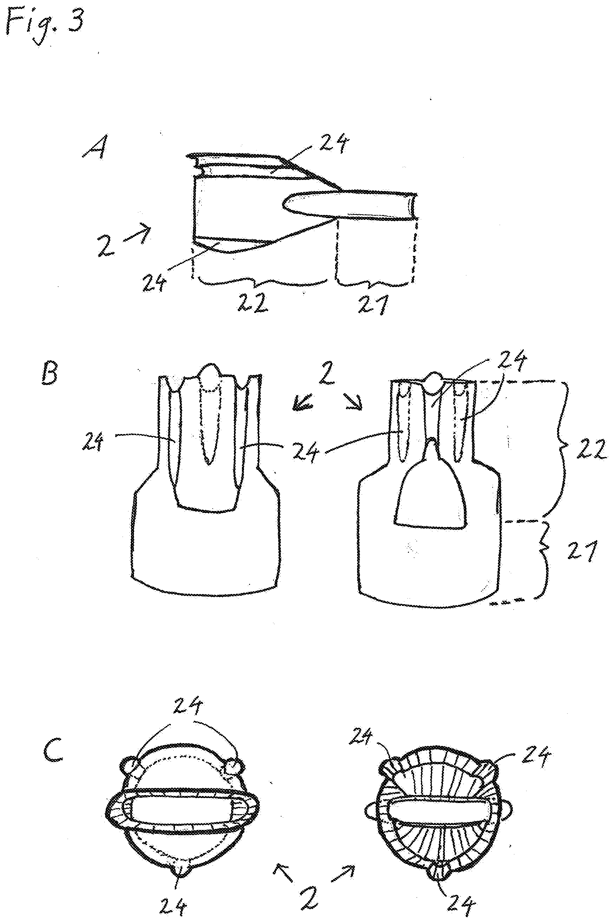

Figur 3 zeigt in drei Teilfiguren eine Fünfseitenansicht des wechselbaren Mundstücks 2 der erfindungsgemäßen Verneblereinheit 1. Teilfigur A ist ein Seitenansicht, die deutlich die Untergliederung des Mundstücks in vorderen, zum Ergreifen mit den Lippen gestalteten, Teil 21 und den zum Aufstecken auf das Anschlussstück 12 der Verneblereinheit 1 passend geformten, hinteren Teil 22. Letzterer geht, wie hier zu sehen ist, mittels ober- wie unterseitig ausgeformter Abschrägungen kontinuierlich in den vorderen Teil über. Ein solcher kontinuierlicher Übergang ist für die Ausbildung einer gleichmäßigen, laminaren Strömung im Inneren des Mundstücks und somit für einen möglichst vollständigen Abtransport des Aerosol-Nebels empfehlenswert. Der untere der Luftkanäle 24 ist an seinem zur Verneblereinheit 1 weisenden Ende so geformt, das er bündig an einen an dieser vorhandenen Flow-Kanal 104 anschließt, so das ein Messabnahmepunkt für einen in einer Steuereinheit befindlichen Druck- oder Durchflusssensor geschaffen wird.
Teilfigur B zeigt das Mundstück in Drauf- (linke Teilabbildung) sowie Untersicht (rechte Teilabbildung). Von den Ausbuchtungen 24, welche im auf das Aufsatzstück 12 aufgesteckten Zustand die Luftkanäle 24 bilden, sind zwei an der Seite und eines mittig an der Unterseite angeformt. Der vordere Teil 21 ist mundgerecht verbreitert. Da das Mundstück wechselbar ist, brauchen weniger Kompromisse eingegangen zu werden, was die genaue Ausgestaltung und Größe dieses Teils 21 angeht, da im Prinzip für jeden Anwender ein passendes Mundstück hergestellt werden kann.
Teilfigur C zeigt Vorder- sowie Rückansicht (linke bzw. rechte Teilabbildung) des wechselbaren Mundstücks vorliegender Erfindung. Deutlich zu sehen ist, das die Ausbuchtungen 24 an ihrer Innenseite im hier dargestellten (von der Verneblereinheit 1) abgenommenen Zustand offen sind, so dass sie sehr leicht gereinigt werden können. Erst beim Aufstecken auf das Anschlussstück 12 wird diese Innenseite durch die Außenseite des, in dieser bevorzugten Ausführungsform im Wesentlichen zylinderförmigen, Anschlussstücks 12 verschlossen und drei, jeweils nur an vorderem und hinterem Ende offene Luftkanäle 24 gebildet.

### Bezugszeichenliste

- 1: Verneblereinheit
- 11: Medikamentenreservoir
- 12: Anschlussstück
- 120: Aerosolkammer
- 13: Kappe
- 101: Aerosolerzeuger
- 102: Dichtring
- 103: Haltestruktur
- 104: Flow-Kanal
- 2: Mundstück
- 21: vorderer Teil
- 22: Hinterer Teil
- 24: Luftkanal, Ausbuchtung

## Patentansprüche

1. Verneblereinheit mit wechselbarem Mundstück umfassend:
- die Verneblereinheit (1), welche in ihrem Innern einen durch Dichtring (102) und Haltestruktur (103) gehaltenen Aerosolerzeuger (101) einschließt und an welcher auf einer Ausgangsseite des Aerosolerzeugers (101) ein Anschlussstück (12) für ein wechselbares Mundstück (2) angeformt ist, und
- das wechselbare Mundstück (2), welches auf die Aufsatzmöglichkeit (12) der Verneblereinheit aufsteckbar ist angeformt ist,
wobei an einer Seite des Mundstücks (2) mindestens ein Luftkanal (24) vorhanden ist, dessen eines Ende in einer stirnseitig durch den Aerosolerzeuger (101) und umfänglich durch das Anschlussstück (12) sowie Mundstück (2) begrenzten Aerosolkammer (120) mündet,
**dadurch gekennzeichnet, dass**
ein anderes Ende mindestens eines der Luftkanäle (24) weitestgehend luftdicht an einen an der Verneblereinheit (1) vorhandenen Flow-Kanal (104) anschließt.

2. Verneblereinheit nach Anspruch 1 **dadurch gekennzeichnet, dass** mehr als ein Luftkanal (24) vorhanden ist.

3. Verneblereinheit nach einem der Ansprüche 1 oder 2,
**dadurch gekennzeichnet, dass** genau drei Luftkanäle (24) vorhanden sind, von denen insbesondere zwei an einer Seite und einer an einer Unterseite des Mundstücks (2) angeformt sind.

4. Verneblereinheit nach einem der vorhergehenden Ansprüche, **dadurch gekennzeichnet, dass** ein Querschnitt des Mundstücks (2) im Wesentlichen zylinderförmig ist.

5. Verneblereinheit nach einem der vorhergehenden Ansprüche, **dadurch gekennzeichnet, dass** das Mundstück (2) aus Kunststoff gefertigt ist.

6. Verneblereinheit nach einem der vorhergehenden Ansprüche, **dadurch gekennzeichnet, dass** ein anderes Ende mindestens eines der Luftkanäle (24) nach Außen offen ist.

7. Verneblereinheit nach einem der vorhergehenden Ansprüche, **dadurch gekennzeichnet, dass** im auf das Anschlussstück (12) der Verneblereinheit (1) aufgesteckten Zustand des Mundstücks (2) die Luftkanäle (24) durch im nichtaufgesteckten Zustand nach innen offene Ausbuchtungen (24) des Mundstücks (2) und eine Außenseite des Anschlussstücks (12) gebildet sind.

8. Verneblereinheit nach einem der vorhergehenden Ansprüche, **dadurch gekennzeichnet, dass** die Aerosolkammer (120) in einem zu der Verneblereinheit (1) gehörigen Teil keine Öffnungen nach außen aufweist.

9. Verneblereinheit nach einem der vorhergehenden Ansprüche, **dadurch gekennzeichnet, dass** in der Verneblereinheit (1) Lufteinlassöffnungen vorhanden sind.

10. Verneblereinheit nach einem der vorhergehenden Ansprüche, **dadurch gekennzeichnet, dass** die Verneblereinheit über ein durch einer Kappe (13) verschlossenes Medikamentenreservoir (11) verfügt.

## Claims

1. Nebulizer unit with exchangeable mouth piece comprising:
- the nebulizer unit (1), which encloses in its interior an aerosol generator (101) held in place by sealing ring (102) and retaining structure (103) and on which, on an exit side of the aerosol generator (101), an attachment piece (12) is formed, and
- the exchangeable mouth piece (2), which is formed to be pluggable onto the attachment facility (12) of the nebulizer unit,
wherein on a side of the mouth piece (2) at least one air channel (24) is present, the end of which opens out into an aerosol chamber (120) delimited facially by the aerosol generator (101) and circumferentially by the attachment piece (12),
**characterized in that**
another end of at least one air channel (24) attaches in an as far as possible air-tight manner to a flow channel (104) present on the nebulizer unit (1).

2. Nebulizer unit according to Claim 1, **characterized in that** more than one air channel (24) is present.

3. Nebulizer unit according to Claim 1 or 2, **characterized in that** exactly three air channels (24) are present, of which in particular two are formed on a side and one on an underside of the mouthpiece (2).

4. Nebulizer unit according to one of the preceding Claims, **characterized in that** a cross section of the mouth piece (2) is substantially cylindrical.

5. Nebulizer unit according to one of the preceding Claims, **characterized in that** the mouth piece (2) is made from plastic.

6. Nebulizer unit according to one of the preceding Claims, **characterized in that** another end of at least one air channel (24) is open to the exterior.

7. Nebulizer unit according to one of the preceding Claims, **characterized in that** in the state of the mouth piece (2), where it is plugged onto the attachment piece (12) of the nebulizer unit (1), the air channels (24) are constituted by salients (24), which, in the unplugged state of the mouth piece (2), are open on an inner side, and an outer face of the attachment piece (12).

8. Nebulizer unit according to one of the preceding Claims, **characterized in that** the aerosol chamber (120) has, in a part belonging to the nebulizer unit (1), no openings to the exterior.

9. Nebulizer unit according to one of the preceding Claims, **characterized in that** in the nebulizer unit (1) air inlets are present.

10. Nebulizer unit according to one of the preceding Claims, **characterized in that** the nebulizer unit possesses a drug reservoir (11) closed by a lid (13).

## Revendications

1. Unité de nébuliseur avec embout buccal pouvant être changé comprenant :
- l'unité de nébuliseur (1), qui comprend à l'intérieur un générateur d'aérosol (101) tenu par une bague d'étanchéité (102) et une structure de maintien (103) et sur laquelle, sur un côté de sortie du générateur d'aérosol (101), est formée une pièce de raccordement (12) pour un embout buccal (2) pouvant être changé, et
- l'embout buccal (2) pouvant être changé, qui est formé de manière à pouvoir être branché sur la pièce de réception (12) de l'unité de nébuliseur,
sachant que sur un côté de l'embout buccal (2) se trouve au moins un conduit d'air (24), dont une extrémité débouche dans une chambre d'aérosol (120) limitée du côté frontal par le générateur d'aérosol (101) et à sa périphérie par la pièce de raccordement (12) ainsi que l'embout buccal (2),
**caractérisée par le fait que**
une autre extrémité d'au moins un des conduits d'air (24) étant raccordée d'une manière largement étanche à l'air à un conduit de flux (104) présent sur l'unité de nébuliseur (1).

2. Unité de nébuliseur selon la revendication 1, **caractérisée par le fait qu'**il y a plus d'un conduit d'air (24).

3. Unité de nébuliseur selon une des revendications 1 ou 2, **caractérisée par le fait qu'**il y a exactement trois conduits d'air (24), parmi lesquels notamment deux sont formés sur un côté et un sur une face inférieure de l'embout buccal (2).

4. Unité de nébuliseur selon une des revendications précédentes, **caractérisée par le fait qu'**une section de l'embout buccal (2) est pour l'essentiel de forme cylindrique.

5. Unité de nébuliseur selon une des revendications précédentes, **caractérisée par le fait que** l'embout buccal (2) est réalisé en plastique.

6. Unité de nébuliseur selon une des revendications précédentes, **caractérisée par le fait qu'**une autre extrémité au moins d'un des conduits d'air (24) est ouverte vers l'extérieur.

7. Unité de nébuliseur selon une des revendications précédentes, **caractérisée par le fait que** lorsque l'embout buccal (2) est branché sur la pièce de raccordement (12) de l'unité de nébuliseur (1), les conduits d'air (24) sont formés par des renflements (24) de l'embout buccal (2) ouverts vers l'intérieur lorsqu'ils ne sont pas branchés, et une face extérieure de la pièce de raccordement (12).

8. Unité de nébuliseur selon une des revendications précédentes, **caractérisée par le fait que** la chambre d'aérosol (120) ne présente pas d'ouvertures vers l'extérieur dans une partie appartenant à l'unité de nébuliseur (1).

9. Unité de nébuliseur selon une des revendications précédentes, **caractérisée par le fait qu'**il y a des ouvertures d'admission d'air dans l'unité de nébuliseur (1).

10. Unité de nébuliseur selon une des revendications précédentes, **caractérisée par le fait que** l'unité de nébuliseur dispose d'un réservoir de médicaments (11) fermé par un capuchon (13).
